# EUROPEAN PATENT APPLICATION

(11) **EP 2 471 467 A1**
(43) Date of publication of application: **04.07.2012**
(21) Application number: 10811867.0
(22) Date of filing: 24.08.2010
(51) Int. Cl.: A61B 17/06

(54) **MEDICAL SUTURE NEEDLE**

(30) Priority: 28.08.2009 JP 2009198666
(71) Applicant: Mani, Inc., Tochigi 321-3231 (JP)
(72) Inventor: KATO Kazuaki, Utsunomiya-shi Tochigi 321-3231 (JP)
(74) Representative: Patentanwälte Westphal, Mussgnug & Partner
(86) International application number: PCT/JP2010/064305
(87) International publication number: WO 2011/024817

(57) **Abstract**

The limit of crush or bend occurring at a needle tip is extended or the strength of a needle tip portion is improved without having a bad influence on operation performed by a medical doctor in a suture surgery. The medical suture needle (A) wherein the needle tip (1) to be thrust into a biomedical tissue is formed at the tip is **characterized in that** the dimension (d) of the needle tip (1) in the direction of the thickness of the material of the medical suture needle has a value not larger than 5% of the thickness (D) of the material of the medical suture needle.

## Description

### Technical Field

The present invention relates to a medical suture needle capable of retaining low thrust resistance while realizing high strength.

### Background Art

The medical suture needles used when suturing biomedical tissue include those called round needles and those called angular needles. In the round needles, the cross-section of the needle tip portion extending to the body portion is circular, while the cross section of the body portion may be circular, triangular, rectangular, planar or the like. The angular needles have a polygonal shape, including a triangular shape and cutting blades are formed at multiple ridges. The round needles also include blunt needles in which the leading end is blunt and after the biomedical tissue is pierced using the needle tip, the blunt needle penetrates the biomedical tissue so as to extend the tissue. In addition, with the angular needle, after the biomedical tissue is pierced by the needle tip, the angular needle is used to cut open the tissue as it penetrates the tissue. Thus an optimal medical suture needle may be selectively used in accordance with the characteristics of the site to be sutured or other conditions.

The medical suture needle includes: a needle tip portion that has a sharp pointed end that is first thrust into the biomedical tissue; a body portion which is the thickest portion and includes a proximal end portion which has a hole for fitting suture thread at its rear end; a taper portion that is formed between the needle tip portion and the body portion and whose thickness gradually increases from the pointed end to the body portion. In the medical suture needle that is formed in this manner, there is a tendency for a great resistance to be generated when the needle is thrust into the biomedical tissue, and then after the needle has been thrust into the biomedical tissue the resistance decreases and then increases again as the thickness increases. This type of change in thrust resistance is particularly remarkable in the round needle.

In the prior art development has generally progressed from the perspective of what can be done to reduce resistance when the medical suture needle is initially thrust into the biomedical tissue and thereby lessen the work of the doctor. In this type of medical suture needle, how the needle tip is processed as a sharp pointed end is important.

A medical suture needle has been proposed which is for suturing non-biomedical tissue such as artificial blood vessels formed by subjecting polytetrafluoroethylene to a special stretching process. In this medical suture needle, the thrust resistance with respect to the artificial blood vessel is reduced (See Patent Literature 1). The medical suture needle disclosed in Patent Literature 1 obtains and maintains favorable thrusting properties by being formed such that the needle tip is pointed and there is an enlarged portion in which the cross-sectional area from the needle tip to the body portion is enlarged and the axial direction length of the enlarged portion is nine times or more the thickness of the body portion and also 2/3 or less of the total length of the suture needle.

There has also been proposed, a medical suture needle that was developed in view of preventing medical accidents that can occur due to the needle tip piercing through the surgical gloves when the diseased site is being sutured (See Patent Literature 2). The medical suture needle disclosed in Patent Literature 2 is excellent in safety and operating properties by being formed so as to have a blunt needle tip where the ratio (D2/D1) of the thickness of the needle tip (D2) to the thickness of the main body of the suture needle (D1) is in a range from 0.04 to 0.30.

### Citation List

### Patent Literature

Patent Literature 1: Japanese Patent Application Publication No. 5-60746
Patent Literature 2: Japanese Patent Application Laid-Open No. 6-296615

### Summary of Invention

### Technical Problem

In the medical suture needle disclosed in Patent Literature 1 in which thrust resistance is reduced by making the needle tip sharp and pointed, the cross-sectional area of the needle tip portion is significantly reduced and the strength with respect to crushing and bending is low. As a result, there is a problem in that the needle tip portion deforms easily when an external force is applied.

That is to say, the medical suture needle in which the needle tip is sharp and pointed is prepared by forming an intermediary material by subjecting one end of a rod-shaped material to pressing and grinding or polishing to form a sharp pointed end and then subjecting the intermediary material to prescribed processing using a number of steps. If while intermediary material passes through the multiple steps, the pointed end comes in contact with other intermediary material or medical equipment, the contact will cause the pointed end to crush or bend.

The manufactured medical suture needles are subjected to total inspection, and at the time of inspection those in which a small amount of crushing or bending occurred are extracted and deemed defective and then subjected to disposal processing. Because the medical suture needle generally has a thickness (body portion thickness) in the range of 0.07 mm to 1.60 mm and the pointed end is formed as a point, and crushing and bending occurs extremely easily when a small force is applied and this is problematic in that disposal processing cost is high.

In the technology of Patent Literature 2, by actively engaging in making the needle tip blunt, it becomes difficult for the needle tip to pierce the surgical gloves and safety is ensured, and by causing the needle tip thrust resistance and the main body thrust resistance to have substantially the same value, no adverse effect is imparted to the doctor in terms of operability. Thus the resistance when the needle tip is thrust into the biomedical tissue becomes larger.

The object of this invention is to provide a medical suture needle in which the limit of crush or bend occurring at the needle tip is extended without any adverse effect on operation at the time of surgery, or in which the strength of the needle tip portion is improved.

### Solution to Problem

The inventors of the present invention noted that despite the fact that the medical suture needle should be formed with a sharp pointed end, when crushing and bending occurs at the needle tip, there is a relationship between the extent to which this crushing and bending has an adverse effect on thrusting properties and the thickness of the material. The inventors focused on this relationship and performed experiments. The results of the experiments revealed that the dimension of the crushing and bending occurring at the needle tip has no great effect up to a particular ratio of material thickness, while if the ratio is exceeded, the effect becomes great and even if the material thickness changes, the ratio is substantially the same.

For this reason, the medical suture needle according to the present invention includes one in which a needle tip for thrusting into biomedical tissue is formed at the leading end and the dimension of the needle tip in the thickness direction of the medical suture needle material is a value not greater than 5% of the thickness of the medical suture needle material.

### Advantageous Effects of Invention

The medical suture needle (also "suture needle" hereinafter) of the present invention enables the resistance when the needle tip is thrust into the biomedical tissue to be smaller than the resistance when the body portion is thrust into the biomedical tissue by setting the dimension of the needle tip in the thickness direction of the medical suture needle material to a value not greater than 5% of the thickness of the material.

In particular, even in the case where crushing and bending occurred in the process of manufacturing the suture needle, if the dimension of the needle tip after crushing (the length in the thickness direction of the medical suture needle material) or the dimension of the needle tip after bending has occurred (length in the thickness direction of the medical suture needle material) is not greater than 5% of the material thickness, a suture needle can be formed which ensures favorable thrusting properties. As a result, when finished product inspection is done, the range of non-defective products is extended.

Furthermore, by presetting the dimension of the needle tip in the thickness direction of the medical suture needle material a value not greater than 5% of the thickness of the material and also close to 5%, ensures favorable thrusting properties without making the leading end of the needle tip sharp and pointed. It also becomes possible to impart strength to resist external forces which act on the needle tip. As a result, crushing and bending which are likely to occur in the manufacturing process are avoided and the number of non-defective products is increased.

### Brief Description of Drawings

Fig. 1 is a diagram showing the suture needle of the present example.
Figs. 2A to 2F are diagrams for explaining the shape of the needle tip.
Figs. 3A to 3F are diagrams showing the relationship measured between the ratio of the thickness of the needle tip to the thickness of the material and the thrust resistance.
Figs. 4A to 4C are diagrams showing the thrust resistance measured when the ratio of the thickness of the needle tip to the thickness of the material is changed.

### Reference Signs List

- A: Suture needle
- 1: Needle tip
- 2: Tapered portion
- 3: Body portion
- 4: Proximal end portion
- 5: Blind hole

### Description of Embodiments

The following describes an exemplary embodiment of the medical suture needle of the present invention. The medical suture needle of the present invention ensures thrusting properties for the biomedical tissue when a doctor performs surgery and it also enables an increase in the dimension of the needle tip in the thickness direction of the medical suture needle material.

In the present invention, there are no particular limitations on the cross-sectional shape of the body portion or the overall shape of the medical suture needle. That is to say, the medical suture needle of this invention may be a curved needle that is curved so as to have a preset curve radius or a straight needle with a substantially linear shape, and may be applied to a suture needle having any shape.

The thickness of the body portion of the medical suture needle of the present invention is standardized and is set within a range from approximately 0.07 mm to approximately 1.60 mm. The thickness of the body portion corresponds to the diameter when the cross sectional area of the needle body is converted to the cross sectional area of a circle and bears no relationship to the cross sectional shape of the body portion. As a result, the thickness of the material corresponds to the thickness of the body portion of the respective suture needles.

There are no particular limitations imposed on the material used for forming the suture needle, and the material may be steel, such as that of piano wire, which is hardened by heat processing, martensitic stainless steel, or austenitic stainless steel. However, for steel and martensitic stainless steel, there is the possibility that rusting may occur at the distribution stage. Thus, although hardening cannot be achieved using heat processing, austenitic stainless steel is preferably used when considering that processing properties are good and rusting does not occur.

### Example 1

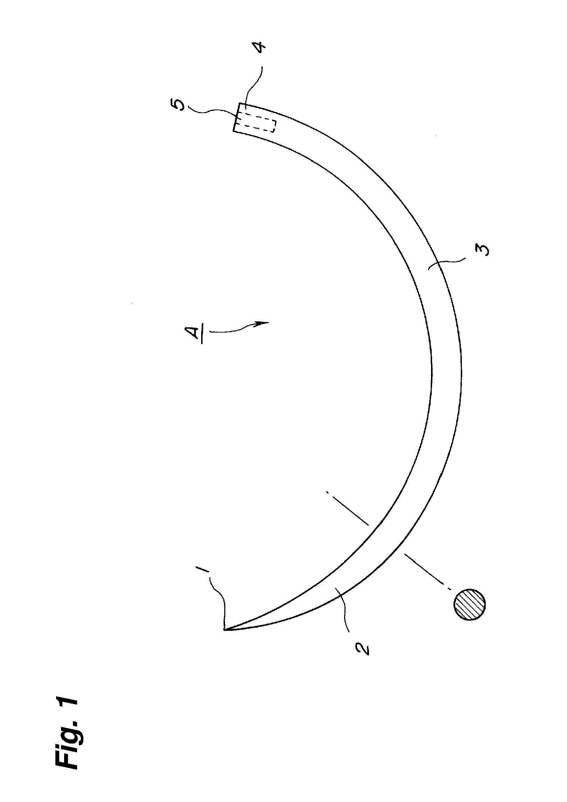

An example of the medical suture needle of the present invention will be described using Fig. 1. The suture needle A shown in Fig. 1 has a needle tip 1 formed at the leading end, a tapered portion 2 formed to be continuous with the needle tip 1, and a body portion 3 continuous with the tapered portion 2. The end portion of the body portion 3 is formed as a proximal end portion 4 which has a blind hole 5 which is for fitting the suture thread which is not shown, into the end surface.

The suture needle A of this example uses material in which austenitic stainless steel wire is subjected to work hardening by performing cold wiredrawing process at a pre-set reduction rate and an austenitic structure is elongated into a fiber. Then, the material of the suture needle A is formed into a round bar. The suture needle A is formed into a curved needle called half circle.

In the suture needle A described above, the cross-sectional shape of the needle tip 1, the taper portion 2 and the body portion 3 is circular and is a socalled round needle. Thus the material of the body portion 3 has substantially the same thickness as the thickness of the material.

The needle tip 1 has a preset shape and may be formed as a pointed end that has a sharp pointed point, or may have a spherical shape. However, there are times when some kind of external force is applied in a step carried out in the manufacturing process and this causes crushing or bending to occur. In particular, in the case where the needle tip 1 is a pointed end, there is a tendency for crushing and bending to occur in the manufacturing process. Thus this invention specifies dimensions that ensure favorable thrusting properties even when crushing and bending of the needle tip 1 occurs.

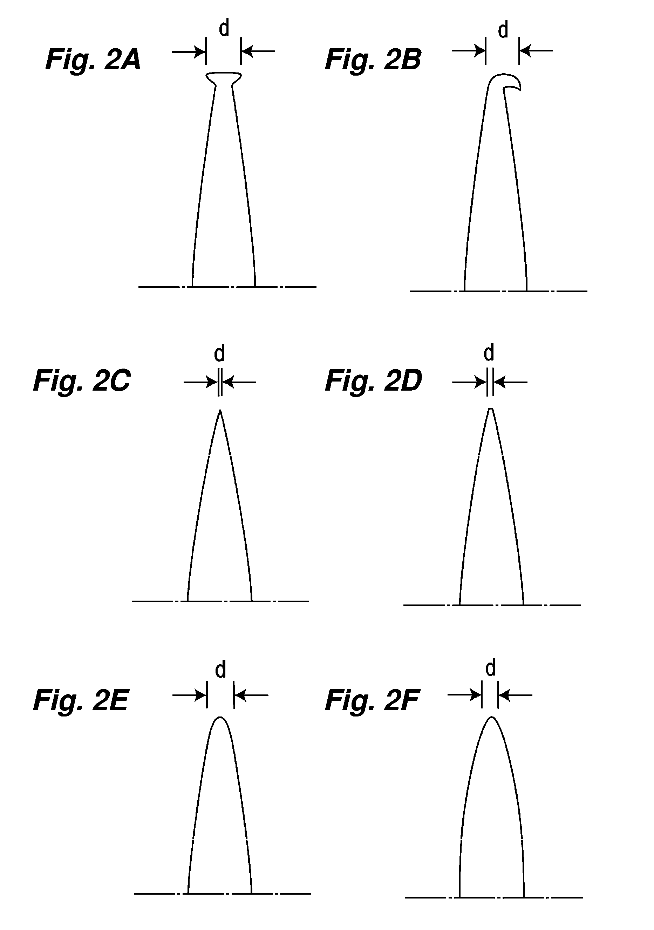

The shape of the needle tip 1 will be described using Figs. 2A to 2F.

Fig. 2A shows the leading end of needle tip 1 in which crushing of dimension d (length in the thickness direction of the surgical suture needle material) has occurred. This type of crushing is often formed when an external force acts straight on the needle tip 1 during the manufacturing process.

Fig. 2B shows the leading end of needle tip 1 in which bending of dimension d (length in the thickness direction of the surgical suture needle material) has occurred. This type of bending is often formed when an external force acts in the horizontal direction with respect to the needle tip 1 during the manufacturing process.

Fig. 2C shows a surgical suture needle in which the leading end of needle tip 1 is formed so as to be sharp and pointed and the dimension d (length in the thickness direction of the surgical suture needle material) of the needle tip 1 is infinitely close to 0. That is to say, a surgical suture needle of this shape would be deemed non-defective.

Fig. 2D shows a surgical suture needle in which the leading end of needle tip 1 has a flat surface of dimension d (length in the thickness direction of the surgical suture needle material). Fig. 2E shows a surgical suture needle in which the leading end of needle tip 1 has a sphere with a diameter (dimension) d (length in the thickness direction of the surgical suture needle material). In Fig. 2F, the leading end of needle tip 1 has a sphere of diameter d and the taper angle from the body portion 3 to the needle tip 1 is large.

The dimension d of each needle tip 1 described above is set to be 5% or less of the thickness D of the material (body portion) forming the suture needle A. By forming a needle tip 1 having this dimension d, thrust properties for the biomedical tissue is ensured and even if crushing or bending occurs at the needle tip 1, the suture needle A can be approved as non-defective.

In the present invention, setting the ratio of dimension d of the needle tip 1 in the thickness direction of the medical suture needle material to a value not greater than 5% of the thickness of the material is accepted based on the results of the experiments. Next, the experiments carried out to obtain the medical suture needle A of the present invention will be described in detail.

In this experiment, multiple test pieces were prepared, each having a needle tip 1 in which the thickness of the material is set and a dimension derived from a preset ratio with respect to the thickness. The energy (thrust resistance in Newtons (N)) required when each sample is used to thrust into the material for thrusting is measured and a determination is made as to whether there is a significant difference from the measured values.

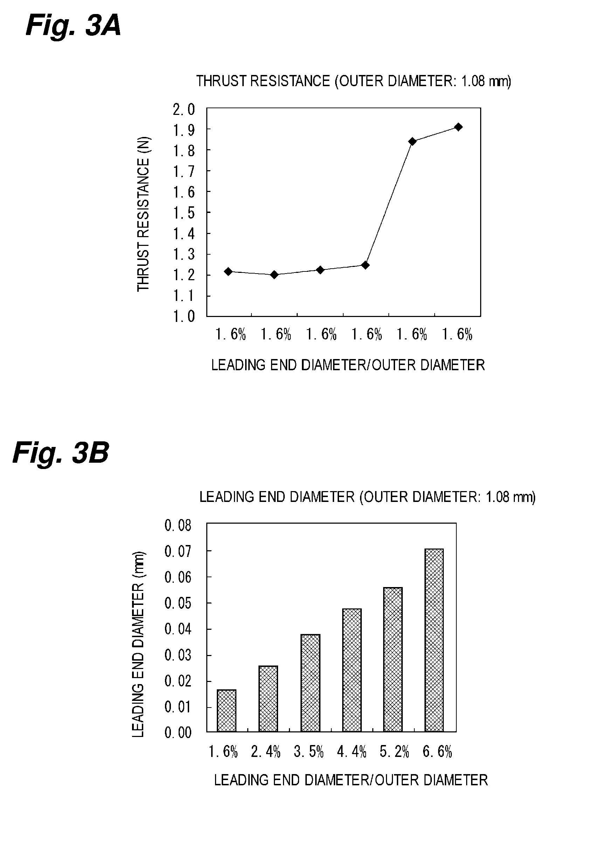

As shown in Figs. 3A to 3F, there are three types of test pieces having material thickness (D) of 1.08 mm, 0.63 mm and 0.33 mm. In the case where the material thickness is 1.08 mm, as shown in Fig. 3B, the dimension d was 0.017 mm and the ratio to material thickness was approximately 1.6%; similarly the dimension d was 0.026 mm and the ratio was approximately 2.4%; the dimension d was 0.038 mm and the ratio approximately 3.5%; the dimension d was 0.048 mm and the ratio approximately 4.4%; the dimension d was 0.056 mm and the ratio approximately 5.2%; the dimension d was 0.071 mm and the ratio approximately 6.6%. In this manner 10 test pieces were prepared for each.

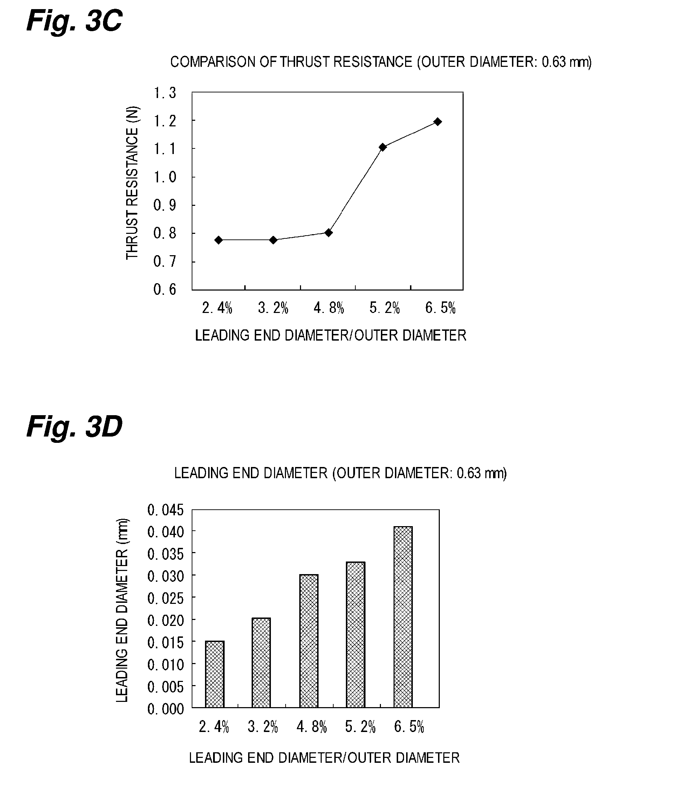

In the case where the material thickness is 0.63 mm, as shown in Fig. 3D, the dimension d of the needle tip 1 was 0.015 mm and the ratio was approximately 2.4%; the dimension d was 0.020 mm and the ratio was approximately 3.2%; the dimension d was 0.030 mm and the ratio approximately 4.8%; the dimension d was 0.033 mm and the ratio approximately 5.2%; the dimension d was 0.041 mm and the ratio approximately 6.5%. In this manner 10 test pieces were prepared for each.

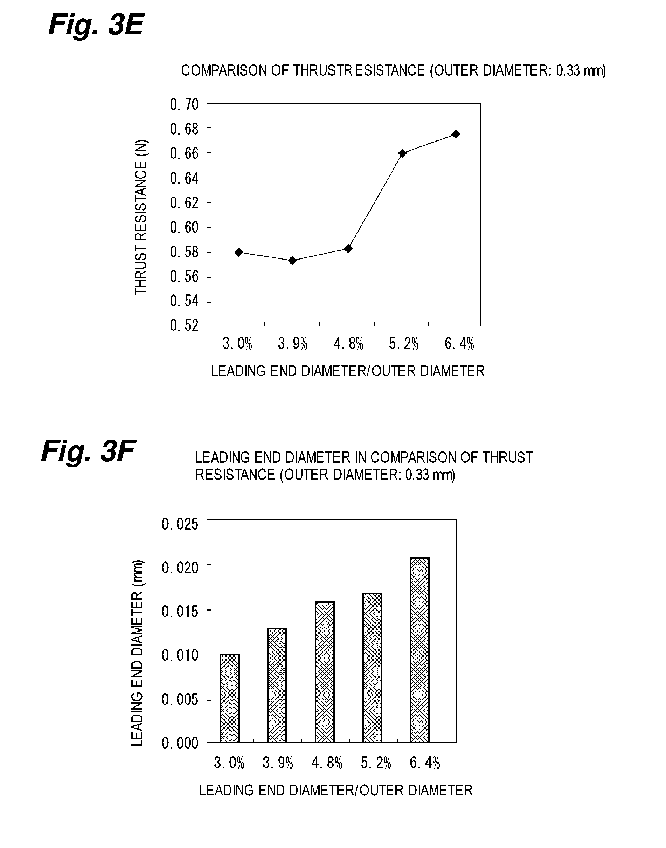

In the case where the material thickness is 0.33 mm, as shown in Fig. 3F, the dimension d of the needle tip 1 is 0.010 mm and the ratio was approximately 3.0%; the dimension d was 0.013 mm and the ratio was approximately 3.9%; the dimension d was 0.016 mm and the ratio approximately 4.8%; the dimension d was 0.017 mm and the ratio approximately 5.2%; the dimension d was 0.021 mm and the ratio approximately 6.4%. In this manner 10 test pieces were prepared for each.

The shape of the needle tip 1 of each of the test pieces described above is spherical. These test pieces were used to thrust into materials for thrusting the suture needle. Porvair with a thickness of 1.10 mm which is usually used when measuring suture needle thrust resistance is used as the material for thrusting the suture needle.

The results of the thrust test in which the test pieces were thrust into the material for thrusting will be described in the following.

Fig. 3A shows the results of measuring the thrust resistance (N) using the test pieces with a material thickness of 1.08 mm. As evident from the figures, for the test pieces in which the ratio of the dimension d of the needle tip 1 to the thickness of the material is from 1.6% to 4.4%, the thrust resistance is concentrated between approximately 1.21 N and approximately 1.25 N, while if the ratio is increased from 4.4% to 5.2%, there is a jump from 1.25 N to 1.9 N which represents a sudden increase of approximately 52%. Subsequently, even when increased to 6.6%, thrust resistance is approximately 1.95 N.

Fig. 3C shows the results of measuring the thrust resistance (N) using the test pieces with a material thickness of 0.63 mm. As evident from the figures, for the test pieces in which the ratio of the dimension d of the needle tip 1 to the thickness of the material is from 2.4% to 4.8%, the thrust resistance is concentrated between approximately 0.79 N and approximately 0.80 N, while if the ratio is increased from 4.8% to 5.2%, there is a jump from 0.8 N to 1.15 N which represents a sudden increase of approximately 43.8%. Subsequently, even when increased to 6.5%, thrust resistance is approximately 1.20 N.

Fig. 3E shows the results of measuring the thrust resistance (N) using the test piece with a material thickness of 0.33 mm. As evident from the figures, for the test pieces in which the ratio of the dimension d of the needle tip 1 to the thickness of the material is from 3.0% to 4.8%, the thrust resistance is between approximately 0.57 N and approximately 0.58 N, while if the ratio is increased from 4.8% to 5.2%, there is a sudden increase from 0.58 N to 0.67 N which is approximately 15.5%. Subsequently, even when increased to 6.4%, thrust resistance is approximately 0.68 N.

The above results of the experiments allowed the following conclusions to be drawn. When the ratio of the dimension d of the needle tip 1 to the thickness D of the material is between 4.8% and 5.2%, a significant difference is generated in that the thrust resistance increases rapidly, and in a range under 4.8%, the thrust resistance is small and the thrust resistance values are substantially equal. However, in the range greater than 5.2%, the thrust resistance increases suddenly and subsequently the increase continues. This trend is not seen in the thickness D of the material and applies only to the ratio of the dimension d to the thickness D of the material.

A test piece was prepared in which the needle tip 1 was crushed so as to forcefully attain the dimension d and a test piece in which the needle tip 1 was bent to forcefully attain the dimension d and the same experiment was carried out on both in order to confirm the results of the experiments above. There was a significant difference when the ratio of the dimension d of the needle tip 1 to the thickness D of the material was between 4.8% and 5.2%.

Next, an experiment was carried out in which the changes in thrust resistance from the needle tip 1 to the body portion 3 in the process of thrusting into the material for thrusting was measured. In this experiment, the thickness of the material was 1.08 mm and a test piece was prepared in which the needle tip 1 had a dimension d of approximately 4.4% of the thickness of the material; a test piece was prepared in which the needle tip 1 had a dimension d of approximately 5.2%; and a test piece was prepared in which the needle tip 1 had a dimension d of approximately 30%. Each of the test pieces was used to thrust into the material for thrusting which was a Porvair with a thickness of 1.10 mm.

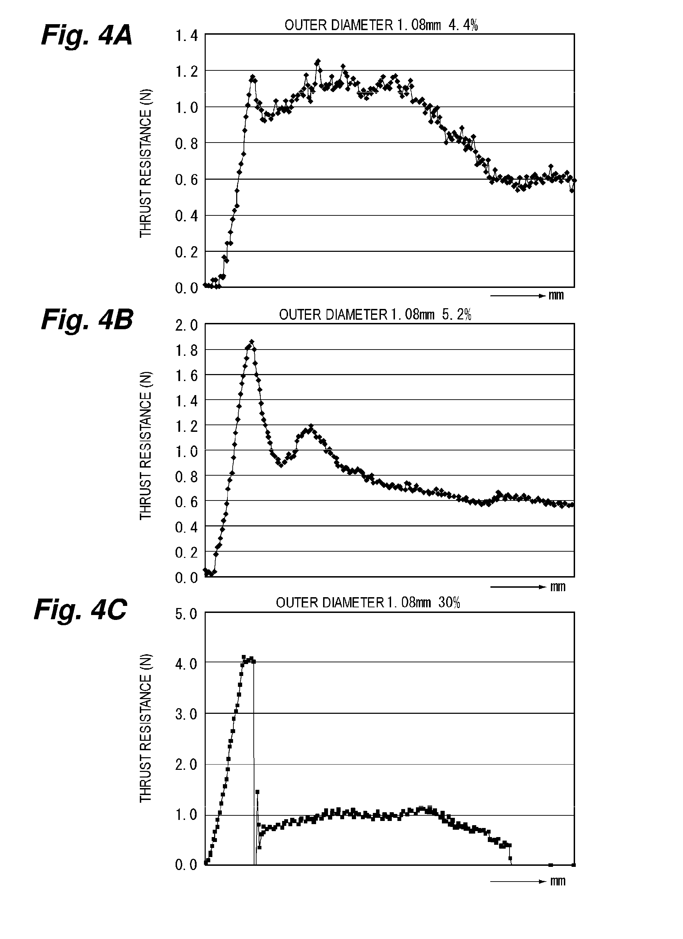

The results of the above experiment will be described using Figs. 4A to 4C. Fig. 4A shows the results of measuring the thrust resistance of the test piece in which the thickness of the material was 1.08 mm and the dimension d of the needle tip 1 is approximately 4.4% of the material thickness. In the figure, the first peak is at the level of approximately 1.19 N and the largest peak is at the level of 1.21 N. Here the first peak is the thrust resistance when the needle tip 1 is thrust into the material for thrusting and the largest peak is the thrust resistance when the body portion is thrust into the material for thrusting. Also because there is little or no difference between both the peaks, no significant difference occurs at the time of the suture surgery.

Fig. 4B shows the results of measuring the thrust resistance of the test piece in which the thickness of the material was 1.08 mm and the dimension d of the needle tip 1 is approximately 5.2% of the material thickness. In the figure, the first peak is at the level of approximately 1.85 N and the next peak is at the level of 1.20 N. Here, the first peak is the thrust resistance when the needle tip 1 is thrust into the material for thrusting and the next peak is the thrust resistance when the body portion is thrust into the material for thrusting. Because there is a difference of approximately 0.7 N between the first peak and the next peak, this difference causes a significant difference at the time of the suture surgery.

Fig. 4C shows the results of measuring the thrust resistance of the test piece in which the thickness of the material was 1.08 mm and the dimension d of the needle tip 1 is approximately 30% of the material thickness. In the figure, the first peak is at the level of approximately 4.1 N and the next peak is at the level of 1.20 N. The first peak is the thrust resistance when the needle tip 1 is thrust into the material for thrusting and the next peak is the thrust resistance when the body portion is thrust into the material for thrusting. There is a difference of approximately 2.9 N between the first peak and the next peak, and this difference causes a significant difference at the time of the suture surgery.

In the results of the above experiments, when the test piece, from the needle tip 1 to the body portion 3, was thrust into the material for thrusting, a significant difference occurs in the test piece in which the ratio of the dimension d of the needle tip 1 to the thickness D of the material is 4.8%, and the test piece in which the ratio is approximately 5.2%. It can therefore be said that the difference gets even larger as the ratio increases.

In order to confirm the results of the experiments above, test pieces were prepared in which the needle tip 1 was crushed so as to forcefully attain the dimension d as well as test pieces in which the needle tip 1 was bent to forcefully attain the dimension d and the same experiment was carried out on both. There was a significant difference when the ratio of the dimension d of the needle tip 1 to the thickness D of the material was between 4.8% and 5.2%.

The results of each of the experiments above led to the conclusion that if the dimension d with respect to the thickness D of the material is 5% or less, favorable thrusting properties can be maintained and even in the case where the suture needle is thrust from the needle tip 1 to the body portion 3, substantially the same level of thrusting properties can be maintained.

For this reason, even in the case where crushing or bending of the needle tip 1 occurs in the manufacturing process, by measuring the dimension d of the bending or crushing at the finished product inspection stage and checking the ratio of the measured dimension d to the thickness of the material D, it becomes possible to approve non-defective products. Thus the yield of the suture needles A can be increased.

In addition, by manufacturing such that the dimension d of the needle tip 1 is preset to a value that is 5% or less of the material thickness D and also close to 5%, the size of the needle tip 1 can be larger and crushing and bending that is likely to occur in the manufacturing process can be avoided.

With regard to the lower limit of the ratio of the dimension d of the needle tip 1 to the material thickness D, a strength with respect to the crushing and bending should be above a certain level, and it is suitable for the value of the ratio to be set such that the ratio of the dimension d of the needle tip 1 to the material thickness D is 5% or less even if crushing and bending occurs.

When the inventors of the present invention carried out experiments, it was found that when the ratio of the needle tip dimension d to the material thickness D is 0.5% or more, the strength to withstand crushing and bending is not problematic and even if crushing or bending occurs, the ratio of the dimension d of the needle tip 1 to the material thickness D is 5% or less. Thus, it can be said that it is effective to set the lower limit of the ratio of the dimension d of the needle tip 1 to the material thickness D so as to be 0.5% or more.

### Industrial Applicability

The medical suture needle of the present invention is advantageous in that it allows increased yield without imposing a large burden on the surgeon at the time of the suture surgery.

## Claims

1. A medical suture needle having a needle tip for thrusting into biomedical tissue formed at a leading end, wherein the dimension of the needle tip in the thickness direction of a medical suture needle material is a value not greater than 5% of the thickness of the medical suture needle.
